(19) Europäisches Patentamt
European Patent Office
Office européen des brevets

(11) Veröffentlichungsnummer: **0 153 274**
**B1**

(12) # EUROPÄISCHE PATENTSCHRIFT

(45) Veröffentlichungstag der Patentschrift: **16.05.90**

(51) Int. Cl.$^5$: **C 07 C 405/00,** A 61 K 31/557

(21) Anmeldenummer: **85730022.2**

(22) Anmeldetag: **07.02.85**

(54) **Neue Carbacycline, Verfahren zu ihrer Herstellung und ihre Verwendung als Arzneimittel.**

(30) Priorität: **10.02.84 DE 3405181**

(43) Veröffentlichungstag der Anmeldung:
**28.08.85 Patentblatt 85/35**

(45) Bekanntmachung des Hinweises auf die Patenterteilung:
**16.05.90 Patentblatt 90/20**

(84) Benannte Vertragsstaaten:
**AT BE CH DE FR GB IT LI LU NL SE**

(56) Entgegenhaltungen:
**EP-A-0 069 692**
**EP-A-0 099 538**
**US-A-4 123 463**

(73) Patentinhaber: **SCHERING AKTIENGESELLSCHAFT Berlin und Bergkamen**
**Müllerstrasse 170/178 Postfach 65 03 11**
**D-1000 Berlin 65 (DE)**

(72) Erfinder: **Skuballa, Werner, Dr.**
**Olwenstrasse 23**
**D-1000 Berlin 28 (DE)**
Erfinder: **Radüchel, Bernd, Dr.**
**Gollanczstrasse 132**
**D-1000 Berlin 28 (DE)**
Erfinder: **Vorbrüggen, Helmut, Prof.**
**Wilkestrasse 7**
**D-1000 Berlin 27 (DE)**
Erfinder: **Nickolson, Robert, Dr.**
**Fuchsienweg 12c**
**D-1000 Berlin 47 (DE)**
Erfinder: **Haberey, Martin, Dr.**
**Neckarsulmer Strasse 15**
**D-1000 Berlin 46 (DE)**
Erfinder: **Loge, Olaf, Dr.**
**Bekassinenweg 37**
**D-1000 Berlin 27 (DE)**
Erfinder: **Stürzebecher, Claus-Steffen, Dr.**
**Kuckucksweg 6**
**D-1000 Berlin 33 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 153 274 B1

**Beschreibung**

Die Erfindung betrifft neue Carbacyclin-Derivate, Verfahren zu ihrer Herstellung sowie ihre Verwendung als Arzneimittel.

Der Vorläufer der Carbacycline, Prostacyclin, wurde 1976 isoliert und im selben Jahr in seiner Struktur aufgeklärt (Prostaglandings *12,* 915, 1976). Als Prostaglandin-Kurzbezeichnung ist für Prostacyclin seit geraumer Zeit die Bezeichnung PG $I_2$ gebräuchlich. Entsprechend werden Carbacycline auch 6a-Carbaprostaglandine-$I_2$ genannt.

Die Nomenklatur der erfindungsgemäßen Verbindungen basiert auf einem Vorschlag von Morton und Brokaw (J. Org. Chem. *44,* 2280 [1979]). Bei der Synthese dieser Verbindungen entstehen stets zwei Doppelbindungsisomere, die durch den Zusatz (5E) oder (5Z) charakterisiert werden.

Auf Grund ihrer biologischen und pharmakologischen Eigenschaften sind Prostacycline und ihre Analoga zur Therapie und Prophylaxe von Thrombosen, Infarkten und anderer Herz-Kreislauf-Erkrankungen geeignet. Häufig ist die Wirkungsdauer dieser Verbindungen für therapeutische Zwecke noch zu kurz. Deshalb haben alle Strukturveränderungen an bekannten $PGI_2$-Derivaten das Ziel, die Wirkungsdauer zu verlängern, die Selektivität der Wirksamkeit zu steigern und gleichzeitig die Wirkungsdosis herabzusetzen.

Aus EP 69 692 sind Carbacycline mit einer primären Alkoholgruppe in 1-Stellung bekannt. Diese Verbindungen senken den Blutdruck und besitzen daneben nür eine schwache cytoprotektive bzw. Magensäuresekretions-hemmende Wirkung. Die Prostaglandine mit einer Alkylacto-Gruppierung am Ende der oberen Seitenkette aus US 4 123 463 wirken cytoprotektiv, sind aber nicht frei von Nebenwirkungen.

Es wurde nun gefunden, daß durch Ersatz der 1-Carboxylgruppe in den 6a-Carbaprostaglandin-$I_2$-derivaten durch eine substituierte Ketongruppe oder eine entsprechende sekundäre Alkoholgruppe eine größere Selektivität, eine bessere Wirksamkeit und eine längere Wirkungsdauer erzielt werden kann.

Die neuen Verbindungen besitzen die für Carbacycline typischen pharmakologischen Eigenschaften, eignen sich jedoch besonders zur Cytoprotektion am Magen, Darm, Herzen, Niere, Leber und Pankreas.

Die Erfindung betrifft Carbacycline der Formel I

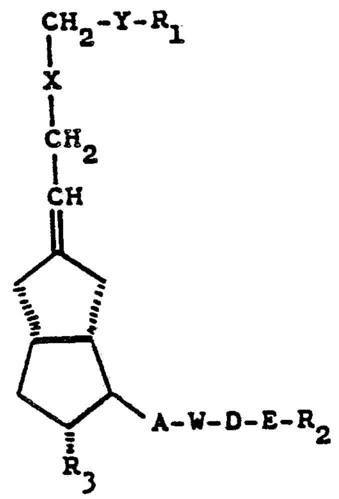

(I),

worin

$R_1$ und $R_2$ Alkylreste mit 1—10 C-Atomen, Cycloalkylgruppen mit 3—10 C-Atomen, Aralkylgruppen mit 6—10 C-Atomen, Phenyl, 1-Naphthyl und 2-Naphthyl, die jeweils durch 1—3 Halogenatome, eine Phenylgruppe, 1—3 Alkylgruppen mit jeweils 1—4 C-Atomen, eine Chlormethyl-, Fluormethyl-, Trifluormethyl-, Carboxyl-, $C_1$—$C_4$-Alkoxy- oder Hydroxygruppe substituiert sein können, oder 5- und 6-gliedrige heterocyclische Gruppen, die wenigstens 1 Heteroatom Stickstoff, Sauerstoff oder Schwefel enthalten, bedeuten,

Y eine Ketogruppe oder eine freie oder funktionell abgewandelte Hydroxymethylengruppe,

X eine —$CH_2$-Gruppe oder ein Sauerstoffatom,

A eine —$CH_2$—$CH_2$—, eine trans-CH=CH— oder —C≡C-Gruppe,

W eine freie oder funktionell abgewandelte Hydroxymethylengruppe oder eine freie oder funktionell abgewandelte

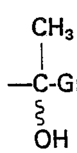

-Gruppe,

wobei die OH-Gruppe α- oder β-ständig sein kann,

D die Gruppe 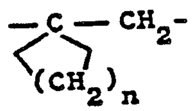 ,

eine geradkettige, gesättigte Alkylengruppe mit 1—10 C-Atomen, eine verzweigte gesättigte oder eine geradkettige oder verzweigte ungesättigte Alkylengruppe mit 2—10 C-Atomen, die gegebenenfalls durch Fluoratome substituiert sein können,

n 1, 2 oder 3 bedeutet,

E eine —C≡C-Gruppe und

$R_3$ eine freie oder funktionell abgewandelte Hydroxygruppe bedeuten.

Die Verbindungen der Formel I stellen sowohl (5E)- als auch (5Z)-Isomere dar. Die Position 5 bezieht sich auf die Carbacyclin-Nomenklatur (s. oben).

Als Alkylgruppen $R_1$ und $R_2$ kommen gerad- und verzweigtkettige, gesättigte Alkylreste mit 1—10 C-Atomen in Frage, die durch Aryl substituiert sein können. Beispielsweise genannt seien Methyl-, Ethyl-, Propyl-, Butyl-, Isobutyl-, tert.-Butyl- Pentyl-, Hexyl-, Heptyl-, Octyl-, Benzyl-.

Die Cycloalkylgruppen $R_1$ und $R_2$ können im Ring 3—10, vorzugsweise 3—6 Kohlenstoffatome enthalten. Die Ringe können durch Alkylgruppen mit 1—4 Kohlenstoffatomen substituiert sein. Beispielsweise seien genannt Cyclopropyl, Cyclobutyl, Cyclopentyl, Cyclohexyl, Methylcyclohexyl und Adamantyl.

Als substituierte bzw. unsubstituierte Arylgruppen $R_1$ und $R_2$ kommen beispielsweise in Betracht: Phenyl, 1-Naphthyl und 2-Naphthyl, die jeweils durch 1—3 Halogenatome, eine Phenylgruppe, 1—3 Alkylgruppen mit jeweils 1—4 C-Atomen, eine Chlormethyl-, Fluormethyl-, Trifluormethyl-, Carboxyl-, $C_1$—$C_4$-Alkoxy- oder Hydroxygruppe substituiert sein können. Bevorzugt ist die Substitution in 3- und 4-Stellung am Phenylring zum Beispiel durch Fluor, Chlor, $C_1$—$C_4$-Alkoxy oder Trifluormethyl oder in 4-Stellung durch Hydroxy.

Als heterocyclische Gruppen $R_1$ und $R_2$ kommen 5- und 6-gliedrige Heterocyclen in Frage, die wenigstens 1 Heteroatom, vorzugsweise Stickstoff, Sauerstoff oder Schwefel enthalten. Beispielsweise seien genannt 2-Furyl, 2-Thienyl 2-Pyridyl, 4-Pyridyl, 3-Furyl, 3-Thienyl u.a.

Als Alkylengruppe D kommen geradkettige mit 1—10 C-Atomen oder verzweigtkettige, gesättigte und ungesättigte Alkylenreste mit 2—10 C-Atomen, vorzugsweise mit 1—5 C-Atomen bzw. 2—5 C-Atomen, in Frage, die gegebenenfalls durch Fluoratome substituiert sein können. Beispielsweise seien genannt: Methylen, Fluormethylen, Ethylen, 1,2-Propylen, Ethylethylen, Trimethylen, Tetramethylen, Pentamethylen, 1-Methyltetramethylen, 1-Methyltrimethylen.

Die Alkylgruppen $R_4$ und $R_5$ stellen geradkettige oder verzweigte gesättigte Alkylgruppen mit 1—5 C-Atomen dar, wie sie bereits für $R_1$ und $R_2$ genannt wurden. $R_5$ als Halogen kann Chlor und Brom, vorzugsweise Chlor, sein.

Die funktionell abgewandelten Hydroxygruppen in Y, W und $R_3$ können Acyloxygruppen mit 2—10 C-Atomen, Benzoyloxy-, Tetrahydropyranyloxy-, Tetrahydrofuranyloxy-, Trimethylsilyloxy-, Tribenzylsilyloxy- oder Dimethyl-tert.-butylsilyloxy sein.

Die Erfindung betrifft ferner ein Verfahren zur Herstellung der Carbacyclin-Derivate der Formel I, dadurch gekennzeichnet, daß man eine Verbindung der Formel II

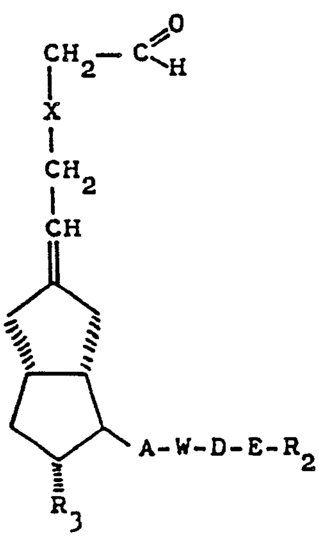

(II),

worin

$R_2$, $R_3$, X, A, W, D und E die oben angegebenen Bedeutungen aufweisen, und freie Hydroxygruppe in W

und $R_3$ gegebenenfalls intermediär geschützt sind, mit metallorganischen Verbindungen der Formel III

$$Me—R_1 \qquad (III)$$

worin

$R_1$ die oben angegebene Bedeutung hat und Me ein Alkalimetall oder Magnesiumhalogenidrest im Sinne eines Grignardreagenzes bedeutet, umsetzt und gegebenenfalls anschließend die 1-Hydroxygruppe oxidiert und/oder geschützte Hydroxygruppen freisetzt.

Die Umsetzung der Verbindungen der Formel II mit der metallorganischen Verbindung der Formel III erfolgt in einem inerten Lösungsmittel oder Lösungsmittelgemisch, wie zum Beispiel Diethylether, Tetrahydrofuran, Dioxan, Toluol, vorzugsweise Tetrahydrofuran oder Diethylether. Die Reaktion wird bei Temperaturen zwischen −100°C und 60°C, vorzugsweise bei −70°C bis 30°C, durchgeführt.

Die Herstellung der für diese Umsetzung benötigten Verbindungen der Formel III erfolgt beispielsweise durch Reaktion der entsprechenden Halogenverbindung mit einem Alkali- oder Erdalkalimetall (z.B. Magnesium) nach bekannten Verfahren.

Die Oxidation der 1-Hydroxygruppe wird nach den dem Fachmann bekannten Methoden vorgenommen. Als Oxidationsmittel können beispielsweise dienen: Pyridiniumdichromat (Tetrahedron Letters, *1979*, 399), Jones-Reagenz (J. Chem. Soc. *1953*, 2555) oder Platin/Sauerstoff (Adv. in Carbohydrate Chem. *17*, 169 (1962) oder Collins-Oxidation. Die Oxidation mit Pyridiniumchromat wird bei Temperaturen von 0°C bis 100°C, vorzugsweise 20°C bis 40°C, in einem gegen das Oxidationsmittel inerten Lösungsmittel, beispielsweise Dimethylformamid, durchgeführt.

Die Oxidation mit Jones-Reagenz wird bei Temperaturen von −40°C bis +40°C, vorzugsweise 0°C bis 30°C, in Aceton als Lösungsmittel ausgeführt.

Die Oxidation mit Platin/Sauerstoff wird bei Temperaturen von 0°C bis 60°C, vorzugsweise 20°C bis 40°C, in einem gegen das Oxidationsmittel inerten Lösungsmittel, wie z.B. Essigester, durchgeführt.

Die funktionelle Abwandlung der freien OH-Gruppen erfolgt nach den dem Fachmann bekannten Methoden. Zur Einführung der Ätherschutzgruppen wird beispielsweise mit Dihydropyran in Methylenchlorid oder Chloroform unter Verwendung eines sauren Kondensationsmittels, wie zum Beispiel p-Toluolsulfonsäure, umgesetzt. Das Dihydropyran wird im Überschuß angewandt, vorzugsweise in der 4- bis 10-fachen Menge des theoretischen Bedarfs. Die Umsetzung ist normalerweise bei 0°C—30°C nach 15 bis 30 Minuten beendet.

Die Einführung der Acylschutzgruppen erfolgt, indem man eine Hydroxy-Verbindung in an sich bekannter Weise mit einem Carbonsäurederivat, wie zum Beispiel Säurechlorid, Säureanhydrid u.a., umsetzt.

Die Freisetzung einer funktionell abgewandelten OH-Gruppe zu den Verbindungen der allgemeinen Formel I erfolgt nach bekannten Methoden. Beispielsweise wird die Abspaltung von Ätherschutzgruppen in einer wäßrigen Lösung einer organischen Säure, wie zum Beispiel Essigsäure, Propionsäure u.a. oder in einer wäßrigen Lösung einer anorganischen Säure, wie zum Beispiel, Salzsäure, durchgeführt. Zur Verbesserung der Löslichkeit wird zweckmäßigerweise ein mit Wasser mischbares inertes organisches Lösungsmittel zugesetzt. Geeignete organische Lösungsmittel sind zum Beispiel Alkohole, wie Methanol und Äthanol, und Äther, wie Dimethoxyäthan, Dioxan und Tetrahydrofuran. Tetrahydrofuran wird bevorzugt angewendet. Die Abspaltung wird vorzugsweise bei Temperaturen zwischen 20°C und 80°C durchgeführt.

Die Abspaltung der Silylätherschutzgruppen erfolgt beispielsweise mit Tetrabutylammoniumfluorid. Als Lösungsmittel sind beispielsweise geeignet Tetrahydrofuran, Diäthyläther, Dioxan, Methylenchlorid usw. Die Abspaltung wird vorzugsweise bei Temperaturen zwischen 0°C und 80°C durchgeführt.

Die Verseifung der Acylgruppen erfolgt beispielsweise mit Alkali- oder Erdalkalicarbonaten oder -hydroxyden in einem Alkohol oder der wäßrigen Lösung eines Alkohols. Als Alkohole kommen aliphatische Alkohole in Betracht, wie zum Beispiel Methanol, Äthanol, Butanol usw., vorzugsweise Methanol. Als Alkalicarbonate und -hydroxyde seien Kalium- und Natriumsalze genannt, bevorzugt sind jedoch die Kaliumsalze. Als Erdalkalicarbonate und -hydroxyde sind beispielsweise geeignet Calciumcarbonat, Calciumhydroxyd und Bariumcarbonat. Die Umsetzung erfolgt bei −10°C bis 70°C, vorzugsweise bei 25°C.

Die als Ausgangsmaterial dienenden Verbindungen der Formel II können beispielsweise hergestellt werden, indem man nach vorherigem Schutz freier Hydroxygruppen einen Methylester der Formel IV

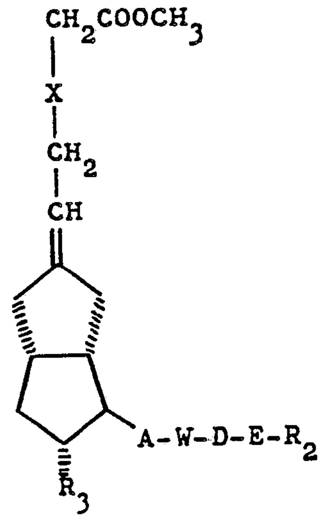

(IV),

worin X, A, W, D, E, $R_2$ und $R_3$ die oben angegebene Bedeutungen aufweisen, mit Lithiumaluminiumhydrid in Diethylether und/oder Tetrahydrofuran gemäß der DE—OS 31 21 155 zum primären Alkohol der Formel V reduziert

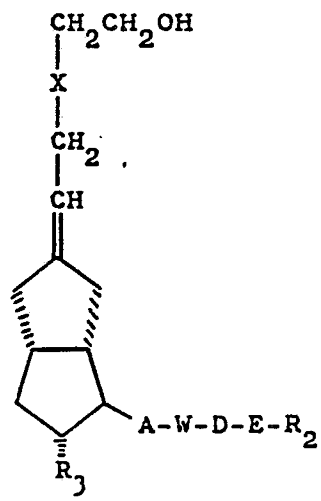

(V)

und anschließend mit Collinsreagenz oder Pyridindichromat oder Pyridiniumchlorochromat oxidiert.

Ein weiterer Weg zur Herstellung der Verbindungen der Formel II besteht in der selektiven Reduktion des Esters der Formel IV mit Diisobutylaluminiumhydrid bei tiefen Temperaturen, vorzugsweise −70°C, in inerten Lösungsmitteln oder Lösungsmittelgemischen, wie beispielsweise Toluol, Tetrahydrofuran oder Methylenchlorid.

Die Methylester der Formel IV können, sofern sie noch nicht bekannt sind, aus den freien Carbonsäuren, die beispielsweise in den DE OS 28 45 770, 30 48 906, 32 04 443, 32 09 702, 33 06 123, 33 06 125 beschrieben sind oder auf analoge Weise hergestellt werden, durch Veresterung mit Diazomethan bei 0°C in Methylenchlorid hergestellt werden. Der gegebenenfalls notwendige Schutz freier Hydroxygruppen in $R_3$ und W kann beispielsweise durch Veretherung mit Dihydropyran oder durch Silylierung erfolgen.

Die Verbindungen der Formel I dieser Erfindung wirken cytoprotektiv. Sie sind prophylaktisch und therapeutisch zur Behandlung von Zellschädigungen geeignet. Folglich stellen die neuen Carbacyclin-Derivate der Formel I wertvolle pharmazeutische Wirkstoffe dar. Darüberhinaus weisen sie, verglichen mit entsprechenden natürlichen Prostaglandinen, eine höhere Spezifität und eine längere Wirksamkeit auf. Im Vergleich zu PG $I_2$ zeichnen sie sich durch größere Stabilität aus. Die hohe Wirkungsspezifität der neuen Carbacycline zeigt sich bei der Untersuchung an glattmuskulären Organen, wie zum Beispiel am Meerschweinchenileum oder an der isolierten Kaninchentrachea, wo eine wesentlich geringere Stimulation zu beobachten ist als bei der Applikation natürlicher Prostaglandine.

Die neuen Carbacyclin-Analoga besitzen einige für Prostacycline typischen Eigenschaften, wie zum Beispiel myocardiale Cytoprotektion, ohne zugleich Schlagvolumen und koronare Durchblutung zu senken, und gastrointestinale Cytoprotektion. Sie eignen sich zur Cytoprotektion der Magen- und Darmschleimhaut, Cytoprotektion in der Leber, Niere und im Pankreas.

Die Dosis der Verbindungen ist 1—1500 µg/kg, wenn sie am menschlichen Patienten verabreicht werden. Die Einheitsdosis für den pharmazeutisch akzeptablen Träger beträgt 0,01—100 mg.

Für die parenterale Verabreichung werden sterile, injizierbare, wässrige oder ölige Lösungen benutzt. Für die orale Applikation sind beispielsweise Tabletten, Dragees oder Kapseln geeignet.

Die Erfindung betrifft damit auch Arzneimittel auf Basis der Verbindungen der Formel I und üblicher Hilfs- und Trägerstoffe, einschließlich Cyclodextrinclathrate.

Die erfindungsgemäßen Wirkstoffe sollen in Verbindung mit den in der Galenik bekannten und üblichen Hilfsstoffen, zum Beispiel zur Herstellung von Cytoprotektiva, dienen.

Beispiel 1

(5E)-(16RS)-2-Descarboxy-2-(1-hydroxyethyl)-16-methyl-18,18,19,19-tetradehydro-6a-carba-prostaglandin-$I_2$

Zu einer Lösung von 513 mg (5E)-(16RS)-2-Descarboxy-2-formyl-16-methyl-18,18,19,19-tetradehydro-6a-carba-prostaglandin-$I_2$-11,15-bis-(tetrahydropyranylether) in 13 ml Diethylether und 13 ml Tetrahydrofuran fügt man bei −70°C 25 ml einer 1,6 molaren etherischen Methyllithiumlösung und rührt 4 Stunden bei 0°C. Anschließend gießt man auf gesättigte Ammoniumchloridlösung, extrahiert mit Ether, wäscht die organische Phase mit Wasser neutral, trocknet über Magnesiumsulfat und dampft im Vakuum ein. Den Rückstand chromatographiert man mit Ether/Hexan (1+1) an Kieselgel. Dabei erhält man 428 mg (5E)-(16RS)-2-Descarboxy-2-(1-hydroxyethyl)-16-methyl-18,18,19,19-tetradehydro-6a-carba-prostaglandin-$I_2$-11,15-bis-(tetrahydropyranylether) als farbloses Öl.

IR($CHCl_3$): 3600, 3450, 2935, 2860, 1602, 1452, 971/cm.

Zur Abspaltung der Schutzgruppen rührt man 428 mg des vorstehend hergestellten Alkohols 16 Stunden bei 25°C mit 38 ml einer Mischung aus Essigsäure/Wasser/Tetrahydrofuran (65/35/10). Man dampft unter Zusatz von Toluol ein und chromatographiert den Rückstand an Kieselgel mit Essigester. Dabei erhält man 295 mg der Titelverbindung als farbloses Öl.

IR: 3600, 3400 (breit), 2925, 2860, 1602, 1450, 970/cm.

Das Ausgangsmaterial für die vorstehende Titelverbindung wird wie folgt hergestellt:

1a)

(5E)-(16RS)-2-Descarboxy-2-formyl-16-methyl-18,18,19,19-tetradehydro-6a-carba-prostaglandin-$I_2$-11,15-bis-(tetrahydropyranylether)

Zu einer Lösung von 2 g (5E)-(16RS)-16-methyl-18,18,19,19-tetradehydro-6a-carba-prostaglandin-$I_2$ in 150 ml Methylenchlorid tropft man bei 0°C eine etherische Lösung von Diazomethan bis zur bleibenden Gelbfärbung. Man rührt 5 Minuten und dampft dann im Vakuum ein. Den so erhaltenen Methylester löst man in 50 ml Methylenchlorid, kühlt auf 0°C ab, fügt 1,8 g Dihydropyran und 20 mg p-Toluolsulfonsäure zu und rührt 30 Minuten bei 0°C. Anschließend verdünnt man mit Ether, schüttelt mit verdünnter Natriumbicarbonat-Lösung, wäscht mit Wasser neutral, trocknet über Magnesiumsulfat und dampft im Vakuum ein. Nach Chromatographie des Rückstandes an Kieselgel erhält man mit Hexan/Ether (1+1) 2,25 g des 11,15-Bistetrahydropyranylethers.

Zur Reduktion löst man 2,25 g des vorstehend erhaltenen Tetrahydropyranylethers in 130 ml Toluol und tropft bei −70°C eine ca. 1,2 molare Lösung von Diisobutylaluminiumhydrid in Toluol zu und rührt 30 Minuten bei −70°C. Anschließend tropft man nacheinander 5 ml Isopropanol und 2,5 ml Wasser zu, rührt 2 Stunden bei 20°C, filtriert und dampft im Vakuum ein. Den Rückstand chromatographiert man mit Hexan/Ether (3+2) an Kieselgel. Dabei erhält man 1,85 g der Titelverbindung als farbloses Öl.

IR: 2940, 2850, 2720, 1720, 1600, 1450, 971/cm.

Beispiel 2

(5E) - (16RS) - 2 - Acetyl - 2 - descarboxy - 16 - methyl - 18,18,19,19 - tetradehydro - 6a - carba - prostaglandin - $I_2$

Eine Lösung von 244 mg (5E) - (16RS) - 2 - Descarboxy - 2 - (1 - hydroxyethyl) - 16 - methyl - 18,18,19,19 - tetradehydro - 6a - carba - prostaglandin - $I_2$ - 11,15 - bis-(tetrahydropyranylether) (aus Beispiel 1) in 7 ml Methylenchlorid fügt man bei 0°C zu einer Lösung von 1 g Collins-Reagenz (Chromsäure-Pyridin-Komplex) in 14 ml Methylenchlorid und rührt 15 Minuten bei 0°C. Anschließend versetzt man mit Ether, filtriert, wäscht das Filtrat nacheinander mit Wasser, 5 %iger Natriumbicarbonatlösung, 10 %iger Schwefelsäure und Wasser, trocknet über Magnesiumsulfat und dampft im Vakuum ein.

Zur Abspaltung der Schutzgruppen rührt man den Eindampfrückstand 16 Stunden bei 25°C mit 24 ml Essigsäure/Wasser/Tetrahydrofuran (65/35/10). Man dampft im Vakuum unter Zusatz von Toluol ein und chromatographiert den Rückstand an Kieselgel mit Essigester und erhält 125 mg der Titelverbindung als farbloses Öl.

IR: 3600, 3340 (breit), 2930, 1710, 1603, 1450, 1430, 1360, 970/cm.

Beispiel 3

(5E) - (16RS) - 2 - Descarboxy - 16,20 - dimethyl - 2 - (1 - hydroxyethyl) - 18,18,19,19 - tetradehydro - 6a - carba - prostaglandin - $I_2$

In Analogie zu Beispiel 1 erhält man aus 590 mg (5E) - (16RS) - 2 - Descarboxy - 16,20 - dimethyl - 2 - formyl - 18,18,19,19 - tetradehydro - 6a - carba - prostaglandin - $I_2$ - 11,15 - bis - (tetrahydropyranylether)

440 mg (5E) - (16RS) - 2 - Descarboxy - 16,20 - dimethyl - 2 - (1 - hydroxyethyl) - 18,18,19,19 - tetradehydro - 6a - carba - prostaglandin - $I_2$ - 11,15 - bis - (tetrahydropyranylether) als farbloses Öl.

IR: 3600, 3440, 2935, 2860, 1602, 1453, 970/cm.

Nach Abspaltung der Schutzgruppen gemäß Beispiel 1 erhält man 310 mg der Titelverbindung als farbloses Öl.

IR: 3600, 3410 (breit), 2927, 2861, 1602, 1451, 971/cm.

Das Ausgangsmaterial für die obige Titelverbindung wird wie folgt hergestellt:

3a)
(5E) - (16RS) - 2 - Descarboxy - 16,20 - dimethyl - 2 - formyl - 18,18,19,19 - tetradehydro - 6a - carba - prostaglandin - $I_2$ - 11,15 - bis - (tetrahydropyranylether)

In Analogie zu Beispiel 1a) erhält man aus 2,5 g (5E) - (16RS) - 16,20 - dimethyl - 18,18,19,19 - tetradehydro - 6a - carba - prostaglandin - $I_2$ 2,1 g der Titelverbindung als farbloses Öl.

IR: 2940, 2852, 2721, 1720, 1602, 1450, 972/cm.

Beispiel 4

(5E) - (16RS) - 2 - Acetyl - 2 - descarboxy - 16,20 - dimethyl - 18,18,19,19 - tetradehydro - 6a - carba - prostaglandin - $I_2$

In Analogie zu Beispiel 2 erhält man aus 320 mg (5E) - (16RS) - 2 - Descarboxy - 16,20 - dimethyl - 2 - (1 - hydroxyethyl) - 18,18,19,19 - tetradehydro - 6a - carba - prostaglandin - $I_2$ - 11,15 - bis - (tetrahydropyranylether) 180 mg der Titelverbindung als farbloses Öl.

IR: 3610, 3330 (breit), 2930, 1710, 1602, 1450, 1430, 971/cm.

Beispiel 5

(5E) - 2 - Descarboxy - 16,16 - dimethyl - 2 - (1 - hydroxyethyl) - 18,18,19,19 - tetradehydro - 6a - carba - prostaglandin - $I_2$

In Analogie zu Beispiel 1 erhält man aus 620 mg (5E) - 2 - Descarboxy - 16,16 - dimethyl - 2 - formyl - 18,18,19,19 - tetradehydro - 6a - carba - prostaglandin - $I_2$ - 11,15 - bis - (tetrahydropyranylether) 465 mg (5E) - 2 - Descarboxy - 16,16 - dimethyl - 2 - (1-hydroxyethyl) - 18,18,19,19 - tetradehydro - 6a - carba - prostaglandin - $I_2$ - 11,15 - bis - (tetrahydropyranylether) als farbloses Öl.

IR: 3600, 3430, 2935, 2860, 1601, 1453, 972/cm.

Nach Abspaltung der Schutzgruppen gemäß Beispiel 1 erhält man 340 mg der Titelverbindung als farbloses Öl.

IR: 3600, 3400 (breit), 2930, 2860, 1601, 1450, 970/cm.

Das Ausgangsmaterial für die obige Titelverbindung wird wie folgt hergestellt:

5a)
(5E) - 2 - Descarboxy - 16,16 - dimethyl - 2 - formyl - 18,18,19,19 - tetradehydro - 6a - carba - prostaglandin - $I_2$ - 11,15 - bis - (tetrahydropyranylether)

In Analogie zu Beispiel 1a erhält man aus 2,3 g (5E) - 16,16 - Dimethyl - 18,18,19,19 - tetradehydro - 6a - carba - prostaglandin - $I_2$ 1,9 g der Titelverbindung als farbloses Öl.

IR: 2940, 2860, 2720, 1718, 1601, 1450, 971/cm.

Beispiel 6

(5E) - 2 - Acetyl - 2 - descarboxy - 16,16 - dimethyl - 18,18,19,19 - tetradehydro - 6a - carba - prostaglandin - $I_2$

In Analogie zu Beispiel 2 erhält man aus 250 mg (5E) - 2 - Descarboxy - 16,16 - dimethyl - 2 - (1 - hydroxyethyl) - 18,18,19,19 - tetradehydro - 6a - carba - prostaglandin - $I_2$ - 11,15 - bis - (tetrahydropyranylether) 125 mg der Titelverbindung als farbloses Öl.

IR: 3600, 3320 (breit), 2932, 1710, 1601, 1450, 970/cm.

Beispiel 7

(5E) - 2 - Descarboxy - 2 - (1 - hydroxyethyl) - 16,16,20 - trimethyl - 18,18,19,19 - tetradehydro - 6a - carba - prostaglandin - $I_2$

In Analogie zu Beispiel 1 erhält man aus 580 mg (5E) - 2 - Descarboxy - 2 - formyl - 16,16,20 - trimethyl - 18,18,19,19 - tetradehydro - 6a - carba - prostaglandin - $I_2$ - 11,15 - bis - (tetrahydropyranylether) 420 mg (5E) - 2 - Descarboxy - 2 - (1 - hydroxyethyl) - 16,16,20 - trimethyl - 18,18,19,19 - tetradehydro - 6a - carba - prostaglandin - $I_2$ - 11,15 - bis - (tetrahydropyranylether) als farbloses Öl.

IR: 3600, 3420, 2936, 2862, 1602, 973/cm.

Nach Abspaltung der Schutzgruppen gemäß Beispiel 1 erhält man 290 mg der Titelverbindung als farbloses Öl.

IR: 3600, 3420 (breit), 2930, 2860, 1602, 1452, 971/cm.

Das Ausgangsmaterial für die obige Titelverbindung wird wie folgt hergestellt:

7a)
(5E) - 2 - Descarboxy - 2 - formyl - 16,16,20 - trimethyl - 18,18,19,19 - tetradehydro - 6a - carba - prostaglandin - $I_2$ - 11,15 - bis - (tetrahydropyranylether)

In Analogie zu Beispiel 1a) erhält man aus 1,9 g (5E) - 16,16,20 - Trimethyl - 18,18,19,19 - tetradehydro - 6a - carba - prostaglandin - $I_2$ 1,54 g der Titelverbindung als farbloses Öl.

IR: 2942, 2861, 2721, 1718, 1602, 1452, 970/cm.

Beispiel 8

(5E) - 2 - Acetyl - 2 - descarboxy - 16,16,20 - trimethyl - 18,18,19,19 - tetradehydro - 6a - carba - prostaglandin - $I_2$

In Analogie zu Beispiel 2 erhält man aus 230 mg (5E) - 2 - Descarboxy - 2 - (1 - hydroxyethyl) - 16,16,20 - trimethyl - 18,18,19,19 - tetradehydro - 6a - carba - prostaglandin - $I_2$ - 11,15 - bis - (tetrahydropyranylether) 110 mg der Titelverbindung als farbloses Öl.

IR: 3600, 3310 (breit), 2930, 1710, 1602, 1451, 971/cm.

Beispiel 9

(5E) - (16RS) - 2 - Descarboxy - 13,14 - didehydro - 16,20 - dimethyl - 2 - (1 - hydroxyethyl) - 18,18,19,19 - tetradehydro - 6a - carba - prostaglandin - $I_2$

In Analogie zu Beispiel 1 erhält man aus 410 mg (5E) - (16RS) - 2 - Descarboxy - 13,14 - didehydro - 16,20 - dimethyl - 2 - formyl - 18,18,19,19 - tetradehydro - 6a - carba - prostaglandin - $I_2$ - 11,15 - bis - (tetrahydropyranylether) 340 mg (5E) - (16RS) - 2 - Descarboxy - 13,14 - didehydro - 16,20 - dimethyl - 2 - (1 - hydroxyethyl) - 18,18,19,19 - tetradehydro - 6a - carba - prostaglandin - $I_2$ - 11,15 - bis - (tetrahydropyranylether) als farbloses Öl.

IR: 3600, 3420, 2936, 2860, 2224/cm.

Nach Abspaltung der Schutzgruppen gemäß Beispiel 1 erhält man 270 mg der Titelverbindung als Öl.

IR: 3605, 3400 (breit), 2930, 2860, 2224/cm.

Das Ausgangsmaterial für die obige Titelverbindung wird wie folgt hergestellt:

9a)
(5E) - (16RS) - 2 - Descarboxy - 13,14 - didehydro - 16,20 - dimethyl - 2 - formyl - 18,18,19,19 - tetradehydro - 6a - carba - prostaglandin - $I_2$ - 11,15 - bis - (tetrahydropyranylether)

In Analogie zu Beispiel 1a) erhält man aus 1,1 g (5E) - (16RS) - 13,14 - Didehydro - 16,20 - dimethyl - 18,18,19,19 - tetradehydro - 6a - carba - prostaglandin - $I_2$ 0,8 g der Titelverbindung als farbloses Öl.

IR: 2940, 2852, 2722, 2224, 1720/cm.

Beispiel 10

(5E) - (16RS) - 2 - Acetyl - 2 - descarboxy - 13,14 - didehydro - 16,20 - dimethyl - 18,18,19,19 - tetradehydro - 6a - carba - prostaglandin - $I_2$

In Analogie zu Beispiel 2 erhält man aus 300 mg (5E) - (16RS) - 2 - Descarboxy - 13,14 - didehydro - 16,20 - dimethyl - 2 - (1 - hydroxyethyl) - 18,18,19,19 - tetradehydro - 6a - carba - prostaglandin - $I_2$ - 11,15 - bis - (tetrahydropyranylether) 175 mg der Titelverbindung als farbloses Öl.

IR: 3600, 3320 (breit), 2930, 2224, 1710/cm.

Beispiel 11

(5E) - (16S) - 2 - Descarboxy - 13,14 - didehydro - 16,20 - dimethyl - 2 - (1 - hydroxyethyl) - 3 - oxa - 18,18,19,19 - tetradehydro - 6a - carba - prostaglandin - $I_2$

In Analogie zu Beispiel 1 erhält man aus 360 mg (5E) - (16S) - 2 - Descarboxy - 13,14 - didehydro - 16,20 - dimethyl - 2 - formyl - 3 - oxa - 18,18,19,19 - tetradehydro - 6a - carba - prostaglandin - $I_2$ - 11,15 - bis - (tetrahydropyranylether) 270 mg (5E) - (16S) - 2 - Descarboxy - 13,14 - didehydro - 16,20 - dimethyl - 2 - (1 - hydroxyethyl) - 3 - oxa - 18,18,19,19 - tetradehydro - 6a - carba - prostaglandin - $I_2$ - 11,15 - bis - (tetrahydropyranylether) als farbloses Öl.

IR: 3610, 3300 (breit), 2932, 2222/cm.

Nach Abspaltung der Schutzgruppen gemäß Beispiel 1 erhält man 180 mg der Titelverbindung als farbloses Öl.

IR: 3600, 3300 (breit), 2932, 2863, 2222/cm.

Das Ausgangsmaterial für die obige Titelverbindung wird wie folgt hergestellt:

11a)
(5E) - (16S) - 2 - Descarboxy - 13,14 - didehydro - 16,20 - dimethyl - 2 - formyl - 3 - oxa - 18,18,19,19 - tetradehydro - 6a - carba - prostaglandin - $I_2$ - 11,15 - bis - (tetrahydropyranylether)

In Analogie zu Beispiel 1a) erhält man aus 0,8 g (5E) - (16S) - 13,14 - Didehydro - 16,20 - dimethyl - 3 - oxa - 18,18,19,19 - tetradehydro - 6a - carba - prostaglandin - $I_2$ 0,6 g der Titelverbindung als farbloses Öl.

IR: 2937, 2853, 2727, 2222, 1719/cm.

Beispiel 12

(5E) - (16S) - 2 - Acetyl - 2 - descarboxy - 13,14 - didehydro - 16,20 - dimethyl - 3 - oxa - 18,18,19,19 - tetradehydro - 6a - carba - prostaglandin - $I_2$

In Analogie zu Beispiel 2 erhält man aus 180 mg (5E) - (16S) - 2 - Descarboxy - 13,14 - didehydro - 16,20 - dimethyl - 2 - (1 - hydroxyethyl) - 3 - oxa - 18,18,19,19 - tetradehydro - 6a - carba - prostaglandin - $I_2$ - 11,15 - bis - (tetrahydropyranylether) 85 mg der Titelverbindung als farbloses Öl.

IR: 3605, 3320 (breit), 2932, 2222, 1713/cm.

Beispiel 13

(5E) - (16RS) - 2 - Descarboxy - 2 - (1 - hydroxypentyl) - 16 - methyl - 18,18,19,19 - tetradehydro - 6a - carba - prostaglandin - $I_2$

In einer Lösung von 450 mg (5E) - (16RS) - 2 - Descarboxy - 2 - formyl - 16 - methyl - 18,18,19,19 - tetradehydro - 6a - carba - prostaglandin - $I_2$ - 11,15 - bis - (tetrahydropyranylether) in 10 ml Diethylether und 10 ml Tetrahydrofuran fügt man bei −70°C 2,2 ml einer 1,6 molaren Butyllithiumlösung in Hexan und rührt 5 Stunden bei 0°C. Anschließend gießt man auf gesättigte Ammoniumchloridlösung, extrahiert mit Ether, wäscht die organische Phase mit Wasser neutral, trocknet über Magnesiumsulfat und dampft im Vakuum ein. Den Rückstand chromatographiert man mit Hexan/Ether (6+4) an Kieselgel. Dabei erhält man 312 mg (5E) - (16RS) - 2 - Descarboxy - 2 - (1 - hydroxypentyl) - 16 - methyl - 18,18,19,19 - tetradehydro - 6a - carba - prostaglandin - $I_2$ - 11,15 - bis - (tetrahydropyranylether) als farbloses Öl.

IR: 3610, 3450 (breit), 2940, 2860, 1602, 1452, 972/cm.

Zur Abspaltung der Schutzgruppen rührt man 312 mg des vorstehend hergestellten Alkohols 16 Stunden bei 25°C mit 28 ml einer Mischung aus Essigsäure/Wasser/Tetrahydrofuran (65/35/10). Man dampft im Vakuum unter Zusatz von Toluol ein und chromatographiert den Rückstand an Kieselgel mit Essigester. Dabei erhält man 190 mg der Titelverbindung als farbloses Öl.

IR: 3600, 3400 (breit), 2935, 2860, 1601, 972/cm.

Beispiel 14

(5E) - (16RS) - 2 - Descarboxy - 16 - methyl - 2 - valeryl - 18,18,19,19 - tetradehydro - 6a - carba - prostaglandin - $I_2$

Zu einer Lösung von 1 g Collins-Reagenz (Chromsäure-Pyridin-Komplex) in 14 ml Methylenchlorid fügt man bei 0°C eine Lösung aus 235 mg (5E) - (16RS) - 2 - Descarboxy - 2 - (1 - hydroxypentyl) - 16 - methyl - 18,18,19,19 - tetradehydro - 6a - carba - prostaglandin - $I_2$ - 11,15 - bis - (tetrahydropyranylether) (aus Beispiel 17) und rührt 20 Minuten bei 0°C. Anschließend versetzt man mit Ether, filtriert, schüttelt das Filtrat nacheinander mit Wasser, 5 %iger Natriumbicarbonatlösung, 10 %iger Schwefelsäure und Wasser, trocknet über Magnesiumsulfat und dampft im Vakuum ein. Zur Abspaltung der Schutzgruppen rührt man den Eindampfrückstand 16 Stunden bei 25°C mit 20 ml Essigsäure/Wasser/Tetrahydrofuran (65/35/10). Man dampft im Vakuum unter Zusatz von Toluol ein und chromatographiert den Rückstand an Kieselgel mit Essigester und erhält 110 mg der Titelverbindung als farbloses Öl.

IR: 3600, 2940, 2870, 1710, 970/cm.

**Patentansprüche**

1. Carbacycline der Formel I

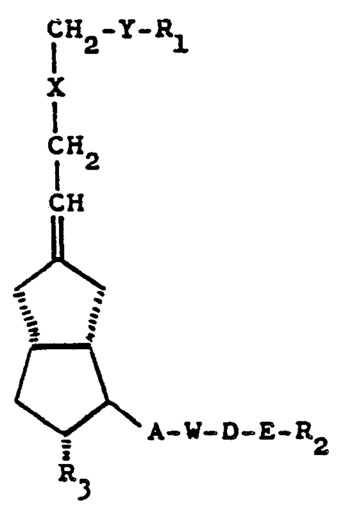

(I),

worin

$R_1$ und $R_2$ Alkylreste mit 1—10 C-Atomen, Cycloalkylgruppen mit 3—10 C-Atomen, Aralkylgruppen mit

6—10 C-Atomen, Phenyl, 1-Naphthyl und 2-Naphthyl, die jeweils durch 1—3 Halogenatome, eine Phenylgruppe, 1—3 Alkylgruppen mit jeweils 1—4 C-Atomen, eine Chlormethyl-, Fluormethyl-, Trifluormethyl-, Carboxyl-, $C_1$—$C_4$-Alkoxy- oder Hydroxygruppe substituiert sein können, oder 5- und 6-gliedrige heterocyclische Gruppen, die wenigstens 1 Heteroatom Stickstoff, Sauerstoff oder Schwefel enthalten, bedeuten,

Y eine Ketogruppe oder eine freie oder funktionell abgewandelte Hydroxymethylengruppe,

X eine $—CH_2$-Gruppe oder ein Sauerstoffatom,

A eine $—CH_2—CH_2—$, eine trans-CH=CH— oder —C≡C-Gruppe,

W eine freie oder funktionell abgewandelte Hydroxymethylengruppe oder eine freie oder funktionell abgewandelte

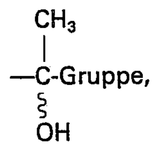

-Gruppe,

wobei die OH-Gruppe α- oder β-ständig sein kann,

D die Gruppe 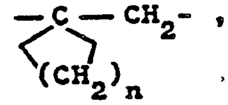 ,

eine geradkettige, gesättigte Alkylengruppe mit 1—10 C-Atomen, eine verzweigte gesättigte oder eine geradkettige oder verzweigte ungesättigte Alkylengruppe mit 2—10 C-Atomen, die gegebenenfalls durch Fluoratome substituiert sein können,

n 1, 2 oder 3 bedeutet,

E eine —C≡C-Gruppe und

$R_3$ eine freie oder funktionell abgewandelte Hydroxygruppe bedeuten.

2. Verfahren zur Herstellung der Carbacyclin-Derivate der Formel I, dadurch gekennzeichnet, daß man eine Verbindung der Formel II

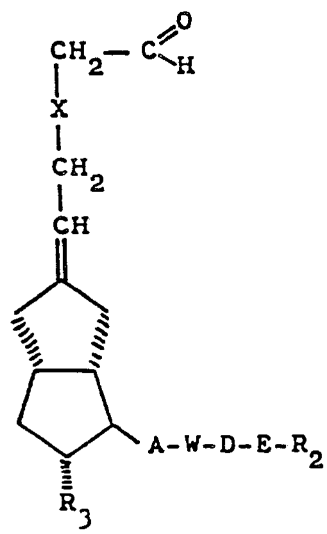

(II),

worin

$R_2$, $R_3$, X, A, W, D und E die oben angegebenen Bedeutungen aufweisen, und freie Hydroxygruppe in W und $R_3$ gegebenenfalls intermediär geschützt sind, mit metallorganischen Verbindungen der Formel III

$$Me—R_1 \quad \text{(III)}$$

worin

$R_1$ die oben angegebene Bedeutung hat und Me ein Alkalimetall oder Magnesiumhalogenidrest im Sinne eines Grignardreagenzes bedeutet, umsetzt und gegebenenfalls anschließend die 1-Hydroxygruppe oxidiert und/oder geschützte Hydroxygruppen freisetzt.

3. Arzneimittel, bestehend aus einer oder mehreren Verbindungen des Anspruchs 1 oder einem Cyclodextrinclathrat einer Verbindung sowie üblichen Hilfs- und Trägerstoffen.

4. (5E) - (16RS) - 2 - Acetyl - 2 - descarboxy - 16 - methyl - 18,18,19,19 - tetradehydro - 6a - carbaprostaglandin - $I_2$.

5. (5E) - 2 - Acetyl - 2 - descarboxy - 16,16,20 - trimethyl - 18,18,19,19 - tetradehydro - 6a - carbaprostaglandin - $I_2$.

**Revendications**

1. Carbacyclines répondant à la Formule I

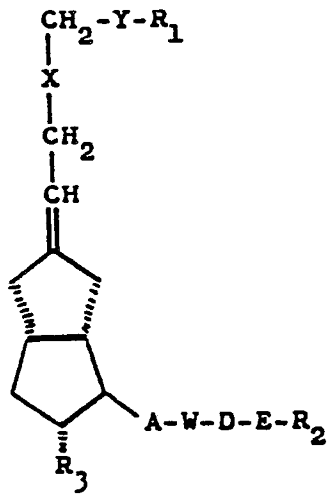

(I),

dans laquelle

$R_1$ et $R_2$ représentent chacun un radical alkyle contenant de 1 à 10 atomes de carbone, un radical cycloalkyle contenant de 3 à 10 atomes de carbone, un radical aralkyle contenant de 6 à 10 atomes de carbone, un phényle, un naphtyle-1 ou un naphtyle-2, (qui peuvent porter chacun de 1 à 3 atomes d'halogènes, un radical phényle, de 1 à 3 radicaux alkyles contenant chacun de 1 à 4 atomes de carbone, ou un radical chlorométhyle, fluorométhyle, trifluorométhyle, carboxy, alcoxy en $C_1$—$C_4$ ou hydroxy), ou un radical hétérocyclique à 5 ou 6 maillons qui contient au moins un hétéroatome pris dans l'ensemble constitué par l'azote, l'oxygène et le soufre,

Y représente un radical carbonyle ou un radical hydroxy-méthylène libre ou sous la forme d'un dérivé fonctionnel,

X représente un radical —$CH_2$— ou un atome d'oxygène,

A représente un radical —$CH_2$—$CH_2$—, un radical —CH=CH— trans ou un radical —C≡C—,

W représente un radical hydroxy-méthylène libre ou sous la forme d'un dérivé fonctionnel, ou un radical

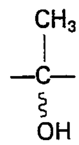

libre ou sous la forme d'un dérivé fonctionnel, le radical OH pouvant avoir la configuration ou,

D représente un radical 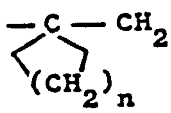

un radical alkylène saturé linéaire qui contient de 1 à 10 atomes de carbone, un radical alkylène en $C_2$—$C_{10}$ saturé et ramifié ou un radical alkylène en $C_2$—$C_{10}$ insaturé et linéaire ou ramifié, qui peuvent éventuellement porter des atomes de fluor,

n est égal à 1, à 2 ou à 3,

E représente un radical —C≡C— et

$R_3$ représente un radical hydroxy libre ou sous la forme d'un dérivé fonctionnel.

2. Procédé pour préparer les carbacyclines de Formule I, procédé caractérisé en ce qu'on fait réagir un composé répondant à la formule II

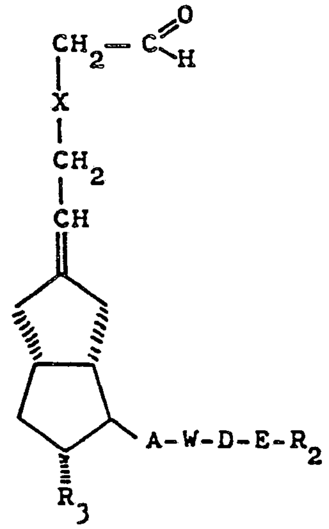

(II),

dans laquelle

$R_2$, $R_3$, X, A, W, D et E ont les significations qui leur ont été données ci-dessus, et un radical hydroxy $R_3$ ou dans W est éventuellement protégé intermédiairement, avec des composés organométalliques répondant à la formule III

$$Me—R_1 \qquad (III)$$

dans laquelle

$R_1$ a la signification qui lui a été donnée plus haut et Me représente un métal alcalin ou un radical d'halogénure de magnésium au sens d'un réactif de Grignard,

et ensuite, éventuellement, on oxyde le radical hydroxy en 1 et/ou on libère des radicaux hydroxy protégés.

3. Médicament constitué d'un ou de plusieurs composés selon la revendication 1 ou d'un clathrate de cyclodextrine d'un composé ainsi que d'adjuvants et excipients usuels.

4. Acétyl - 2 descarboxy - 2 méthyl - 16 tétradéhydro - 18,18,19,19 carba - 6a prostaglandine $I_2$ (5E) - (16RS).

5. Acétyl - 2 descarboxy - 2 triméthyl - 16,16,20 tétradéhydro - 18,18,19,19 carba - 6a prostaglandine $I_2$ (5E).

**Claims**

1. Carbacyclins of the Formula I

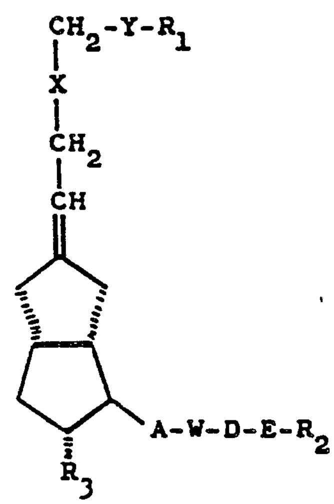

(I),

wherein

$R_1$ and $R_2$ are alkyl radicals having 1—10 carbon atoms, cycloalkyl groups having 3—10 carbon atoms, aralkyl groups having 6—10 carbon atoms, phenyl, 1-naphthyl and 2-naphthyl, each of which can be substituted by 1—3 halogen atoms, a phenyl group, 1—3 alkyl groups each having 1—4 carbon atoms, a

chloromethyl, fluoromethyl, trifluoromethyl, carboxy, $C_1$—$C_4$-alkoxy or hydroxy group, or are 5- and 6-membered heterocyclic groups containing at least one hetero atom nitrogen, oxygen or sulphur,

Y is a keto group or a free or functionally modified hydroxymethylene group,

X is a —$CH_2$— group or an oxygen atom,

A is a —$CH_2$—$CH_2$—, a trans-CH=CH— or —C≡C— group,

W is a free or functionally modified hydroxymethylene group or a free or functionally modified

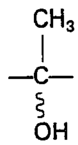 group

wherein the OH group can be in the α- or β- configuration,

D is the group 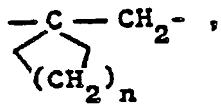 ,

a straight-chain, saturated alkylene group having 1—10 carbon atoms, a branched saturated or a straight-chain or branched unsaturated alkylene group having 2—10 carbon atoms, each of which can optionally be substituted by fluorine atoms,

n is 1, 2 or 3,

E is a —C≡C— group, and

$R_3$ is a free or functionally modified hydroxy group.

2. A process for the preparation of the carbacyclin derivatives of formula I, characterised in that a compound of the formula II

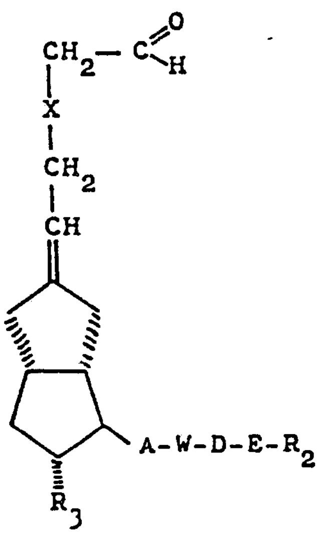

(II),

wherein

$R_2$, $R_3$, X, A, W, D and E are as defined above and free hydroxy groups in W and $R_3$, if desired, are intermediately protected, is reacted with organometallic compounds of formula III

$$Me—R_1 \qquad (III)$$

wherein

$R_1$ is as defined above and Me is an alkali metal or a magnesium halide radical in the sense of a Grignard reagent, and, optionally, subsequently the 1-hydroxy group is oxidised and/or protected hydroxy groups are freed.

3. A medicinal agent consisting of one or more compounds of claim 1 or a cyclodextrin clathrate of a compound and customary auxiliary agents and excipients.

4. (5E) - (16RS) - 2 - acetyl - 2 - decarboxy - 16 - methyl - 18,18,19,19 - tetradehydro - 6a - carbaprostaglandin $I_2$.

5. (5E) - 2 - acetyl - 2 - decarboxy - 16,16,20 - trimethyl - 18,18,19,19 - tetradehydro - 6a - carbaprostaglandin $I_2$.